(19) Europäisches Patentamt
European Patent Office
Office européen des brevets

(11) Publication number: **0 201 871**
**B1**

# (12) EUROPEAN PATENT SPECIFICATION

(45) Date of publication of the patent specification: **10.08.88**

(51) Int. Cl.⁴: **C 07 C 43/12**, C 07 C 41/06

(21) Application number: **86106297.4**

(22) Date of filling: **07.05.86**

## (54) Fluoro-halo-ethers and process to produce them.

(30) Priority: **17.05.85 IT 2078185**

(43) Date of publication of application: **20.11.86 Bulletin 86/47**

(45) Publication of the grant of the patent: **10.08.88 Bulletin 88/32**

(84) Designated Contracting States: **AT BE CH DE FR GB LI NL SE**

(56) References cited:
**DE-A-3 438 934**
**US-A-3 558 721**
**US-A-4 334 105**

(73) Proprietor: **AUSIMONT S.p.A., 31, Foro Buonaparte, I-20121 Milano (IT)**

(72) Inventor: **Guglielmo, Giorgio, Via A.Gramsci 89, I-30035 Mirano/Venezia (IT)**
Inventor: **Gambaretto, Giampaolo, 7, via Torino, I-35142 Padova (IT)**

(74) Representative: **Jaumann, Paolo, Studio Consulenza Brevetti Jaumann Piazza Castello, 2, I-20121- Milano (IT)**

**EP 0 201 871 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1988

## Description

## Background of the Invention

1. Field of the Invention

The present invention relates to fluoro-halo-ethers and to the process to obtain them.

More particularly, the present invention relates to fluoro-halo-ethers suitable to be dehalogenated to the purpose of forming the corresponding fluoro-halogenated vinyl ethers, and to the related process to obtain them.

2. Description of the Prior Art

From the technical literature some types of fluoro-halo-ethers are known, which are advantageously used as hypnotic or anaesthetic agents, and in the phytosanitary and phytopharmaceutical field.

These fluoro-halo-ethers of the prior art are characterized in that both the radicals bonded to the oxygen atom contain hydrogen.

U.S. Patents Nos. 3 557 294; 3 897 502; 3 784 706; 3 764 706; 3 947 595; 3 976 788; 3 943 256; 3 987 111; 4 357 282 disclose these fluoro-halo-ethers and the process for preparing them.

U.S. Patent No. 4 334 105 discloses a particular type of halo-ethers, the characteristic of which is that of having only one reducible group "$CZ_2$" on at least one of the radicals bonded to the oxygen atom. According to said patent, such characteristic of reducibility is achieved when "Z" is either bromine or chlorine, or both of them.

The presence of only one reducible group on each radical does not allow the corresponding vinyl-ethers to be obtained by simple dehalogenating.

The French Patent No. 2 287 432 discloses some fluorinated mono- and di-ethers, which, in the case of monoethers, correspond to the general formula:

$$F - R_f - CF_2 - O - R - H$$

wherein $R_f$ is a perfluorinated aliphatic chain and R is a possibly substituted aliphatic radical.

Also these fluoro-ethers are however unsuitable to undergo a dehalogenating reaction leading to the formation of fluoro-halogenated vinyl-ethers.

The Present Invention

Purpose of the present invention is to provide new fluoro-halo-ethers suitable to be de halogenated to the purpose of forming the corresponding vinyl-ethers.

Further purpose of the present invention is to provide a process for the preparation of fluoro-halo-ethers suitable to be dehalogenated to the purpose of forming the corresponding vinyl-ethers.

It has now been surprisingly found by the present Applicant and this is one object of the present invention, that these purposes are achieved by means of new fluoro-halo-ethers of general formula:

$(R)_nC(F)_m$-O-CAF-$CAF_2$ (I)

wherein A is selected from chlorine and bromine, R is an alkyl, cycloalkyl, aromatic, alkyl monoether or alkyl polyether radical containing from 1 to 20 carbon atoms, partly or completely halogenated with bromine, chlorine, iodine and/or fluorine as substituents, n is an integer selected from 1 and 2, m is an integer equal to 3-n, it being intended that the value n = 2 comprises the compounds in which C is part of a cyclic ring.

In the present invention the fluoro-halo-ethers of general formula (I) wherein A is chlorine and R is either a perhaloalkyl radical containing from 1 to 5 carbon atoms and halogens of the type of fluorine, chlorine and/or bromine, or a perfluoroalkyl monoether containing up to 5 carbon atoms, are preferred.

Examples of fluoro-halo-ethers being the object of the present invention are those having the following formulae:

$CF_3$-$CF_2$-$CF_2$-O-CClF-$CClF_2$
$CF_3$-$CF_2$-O-CClF-$CClF_2$
$CClF_2$-$CF_2$-O-CClF-$CClF_2$
$CCl_2F$-$CF_2$-O-CClF-$CClF_2$
$CCl_3$-$CF_2$-O-CClF-$CClF_2$
$CBrF_2$-$CF_2$-O-CClF-$CClF_2$
$CF_3$-O-$CF_2CF_2$-O-CClF-$CClF_2$
$C_2F_5$-O-$CF_2CF_2$-O-CClF-$CClF_2$
$(CF_3)_2CF$-O-CClF-$CClF_2$

The fluoro-halo-ethers being the object of the present invention have never been described in the technical literature and have the characteristic that both their groups -CAF- are reducible. This characteristic allows the corresponding fluoro-halogenated vinyl-ethers to be obtained by dehalogenation.

According to another aspect of the present invention, the products of formula (I) can be obtained by a synthesis route starting from a fluoro-oxy-halo compound and a halo-olefin according to the reaction scheme:

$$\underset{(II)}{(R)_nC(F)_m\text{-O-F}} + \underset{(III)}{CAF{=}CAF} \rightarrow \underset{(I)}{(R)_nC(F)_mO\text{-}CAF\text{-}CAF_2}$$

wherein A, R, n and m have the same meaning as described hereinabove.

The fluoro-oxy-halo compound having general formula:

$(R)_nC(F)_m$-O-FK15>(II)

can be obtained according to any known process, and in particular according to the process disclosed in Italian Patent Application No. 19 847 A/85.

The compounds being the object of the present invention, having the general formula (I), can be obtained by reacting the fluoro-oxy-halo compound with the halogenated olefin, for example in the gas phase, under atmospheric or slightly superatmospheric pressure, such as, e.g., up to 5 atmospheres, at a temperature comprised within the range of from -80° C to 50° C.

The reaction can be carried out by feeding the reactants as such, or diluted by inert gases or vapours, such as, e.g., $N_2$, He, $CF_4$, $CClF_3$, $CCl_2F_2$, $CHClF_2$, $C_2ClF_5$, $C_2Cl_2F_4$.

In the process being object of the present invention, concentrations of the sum of the reactants comprised within the range of from 1 to 50 % by volume are preferred.

Examples illustrative of compounds having general formula (II) which can be used in the process being one object of the present invention comprise:

fluoro-oxy-pentafluoro-ethane; fluorooxy-2-chloro-tetrafluoro-ethane; fluorooxy-2,2-dichloro-trifluoroethane; fluorooxy-2,2,2-trichloro-difluoroethane; fluorooxy-2-bromo-tetrafluoro-ethane; fluorooxy-heptafluoropropane; fluorooxy-perfluoro-butane; perfluoro-ethoxy-perfluoroethylhypofluorite; perfluoromethoxy-perfluoroethyl-hypofluorite and their mixtures; perfluoroisopropyl-hypofluorite.

In the process being object of the present invention any halogenated olefin of general formula (III) can be used, on condition that both its groups -CAF- are reducible. Illustrative examples of preferred halogenated olefins are: 1,2-difluoro-dichloro-ethylene, 1,2-difluoro-dibromo-ethylene, 1-chloro-2-bromo-difluoro-ethylene.

To the purpose of better understanding the present invention and to show practical embodiments thereof, hereunder some illustrative Examples are reported.

**Example 1**

To the top of a metal tubular reactor of 1 cm of inner diameter and of 1300 cm of length, cooled at -30° C, an $N_2/C_2F_5OF$ gas mixture at 20 % by volume of $C_2F_5OF$ is fed under an absolute pressure of 110 kPa; at a distance of about 300 cm from the top a $C_2ClF_5/CClF{=}CClF$ gas mixture at 20 % by volume of $CClF{=}CClF$ is fed. The flow rate of the two streams is adjusted at 18.7 Nl/h each. The stream leaving the reactor is cooled to -80° C and the condensate is distilled.

After 6 hours of continuous feeding, 72 g is obtained of a fraction at 99 % by weight of $C_2F_5OCClF\text{-}CClF_2$. Yield 25 % by mol.

The product is identified by mass spectrophotometry and has a boiling point of 60 - 62° C.

A sample of the so obtained product has been submitted to a dechlorination process by zinc in dimethylsulphoxide at 40° C, with a yield higher than 90 %. The corresponding fluorovinylether.

$$C_2F_5OCF{=}CF_2$$

obtained has a boiling point of 9° C.

**Example 2**

The test is carried out as in Example 1, but feeding at the reactor top an $N_2/CClF_2\text{-}CF_2OF$ gas mixture at 20 % by volume of $CClF_2\text{-}CF_2OF$.

After 10 hours of continuous feeding and subsequent distillation, 31 g is obtained of product at 99 %, which by mass spectrophotometric analysis has been identified as:

$$CClF_2\text{-}CF_2\text{-}O\text{-}CClF\text{-}CClF_2$$

the product shows a boiling point of 90 - 92° C.

**Example 3**

The test is carried out as in Example 1, but feeding at the reactor top an $N_2/CF_3$-$CF_2$-$CF_2$-OF gas mixture at 20 % by volume of $CF_3$-$CF_2$-$CF_2$-OF.

After 10 hours of continuous feeding and subsequent distillation, 40 g is obtained of product at 99 %, which by mass spectrophotometric analysis has been identified as

$CF_3$-$CF_2$-$CF_2$-O-CClF-$CClF_2$.

**Claims** for the Contracting States: BE, CH, DE, FR, GB, LI, NL, SE

1. Fluoro-halo-ethers of general formula:

$(R)_nC(F)_m$-O-CAF-$CAF_2$

wherein A is selected from chlorine and bromine, R is an alkyl, cycloalkyl, aromatic, alkyl monoether or alkyl polyether radical containing from 1 to 20 carbon atoms, partly or completely halogenated with bromine, chlorine, iodine and/or fluorine as substituents, n is an integer selected from 1 and 2, m is an integer equal to 3-n, wherein the value n 2 comprises the compounds in which C is part of a cyclic ring.

2. Fluoro-halo-ethers according to claim 1, wherein R is a perhaloalkyl radical containing from 1 to 5 carbon atoms.

3. Fluoro-halo-ethers according to claim 2, wherein the perhaloalkyl radical contains halogens of the type of fluorine, chlorine and/or bromine.

4. Fluoro-halo-ethers according to claim 1, wherein R is a perfluoroalkyl monoether radical containing to 5 carbon atoms.

5. Fluoro-halo-ether according to any of foregoing claims, wherein A is chlorine.

6. Fluro-halo-ether according to claim 1, of formula:

$CF_3$-$CF_2$-O-CClF-$CClF_2$

7. Fluoro-halo-ether according to claim 1, of formula:

$CClF_2$-$CF_2$-O-CClF-$CClF_2$

8. Fluor-ohalo-ether according to claim 1, of formula:

$CCl_2F$-$CF_2$-O-CClF-$CClF_2$

9. Fluoro-halo-ether according to claim 1, of formula:

$CCl_3$-$CF_2$-O-CClF-$CClF_2$

10. Fluoro-halo-ether according to claim 1, formula:

$CBrF_2$-$CF_2$-O-CClF-$CClF_2$

11. Fluoro-halo-ether according to claim 1, of formula:

$CF_3$-O-$CF_2CF_2$-O-CClF-$CClF_2$

12. Fluoro-halo-ether according to claim 1, of formula:

$C_2F_5$-O-$CF_2CF_2$-O-CClF-$CClF_2$

13. Fluoro-halo-ether according to claim 1, of formula:

$(CF_3)_2$-CF-O-CClF-$CClF_2$

14. Fluoro-halo-ether according to claim 1, of formula:

$CF_3CF_2CF_2$-O-CClF-$CClF_2$

15. Process for the preparation of the fluoro-halo-ethers according to any of foregoing claims, consisting in reacting a fluoro-oxy-halo compound with a halogenated olefin according to the scheme:

$(R)_nC(F)_m$-O-F + CAF=CAF → $(R)_nC(F)_m$-O-CAF-$CAF_2$

wherein A, R, n and m have the hereinabove reported meaning.

**Claims** for the Connecting State: AT

1. A process for preparing fluoro-halo-ethers of general formula:

$(R)_nC(F)_m$-O-CAF-$CAF_2$

wherein A is selected from chlorine and bromine, R is an alkyl, cycloalkyl, aromatic, alkyl monoether or alkyl polyether radical containing from 1 to 20 carbon atoms, partly or completely halogenated with bromine, chlorine, iodine and/or fluorine as substituents, n is an integer selected from 1 and 2, m is an integer equal to 3-n, wherein the value n 2 comprises the compounds in which C is part of a cyclic ring, said process consisting in reacting a fluoro-oxy-halo compound with a halogenated olefin according to the scheme:

$$(R)_nC(F)_m\text{-O-F} + CAF = CAF \rightarrow (R)_nC(F)_m\text{-O-CAF-}CAF_2$$

wherein A, R, n and m have the hereinabove reported meaning.

2. A process according to claim 1, wherein R is a perhaloalkyl radical containing from 1 to 5 carbon atoms.

3. A process according to claim 2, wherein the perhaloalkyl radical contains halogens of the type of fluorine, chlorine and/or bromine.

4. A process according to claim 1, wherein R is a perfluoroalkyl monoether radical containing up to 5 carbon atoms.

5. A process according to any of foregoing claims wherein A is chlorine.

**Patentansprüche** für die Vertragsstaaten: BE, CH, DE, FR, GR, LI, NL, SE

1. Fluorhalogenäther der allgemeinen Formel:

$$(R)_nC(F)_m\text{-O-CAF-}CAF_2$$

wobei A unter Chlor und Brom gewählt ist, R ein alkylischer, cycloalkylischer, aromatischer, alkylmonoätherischer oder alkylpolyätherischer

Rest mit 1 bis 20 Kohlenstoffatomen ist, welcher teilweise oder vollkommen mit Brom, Chlor, Jod und/oder Fluor Substituenten halogeniert ist, n eine der ganzen Zahlen 1 oder 2 und m die ganze Zahl 3-n bedeutet, wobei der Wert n = 2 solche Verbindungen umfaßt, in welchen C Bestandteil eines cyclischen Ringes ist.

2. Fluorhalogenäher nach Anspruch 1, wobei R ein perhalogenalkylischer Rest mit 1 bis 5 Kohlenstoffatomen ist.

3. Fluorhalogenäther nach Anspruch 2, wobei der perhalogenalkylische Rest Halogene, wie Fluor, Chlor und/oder Brom enthält.

4. Fluorhalogenäther nach Anspruch 1, wobei R ein perfluoralkylmonoätherischer Rest mit bis zu 5 Kohlenstoffatomen ist.

5. Fluorhalogenäther nach einem der vorangehenden Ansprüche, wobei A Chlor ist.

6. Fluorhalogenäther nach Anspruch 1 mit der Formel:

$CF_3\text{-}CF_2\text{-O-CClF-}CClF_2$.

7. Fluorhalogenäther nach Anspruch 1 mit der Formel:

$CClF_2\text{-}CF_2\text{-O-CClF-}CClF_2$.

8. Fluorhalogenäther nach Anspruch 1 mit der Formel:

$CCl_2F\text{-}CF_2\text{-O-CClF-}CClF_2$.

9. Fluorhalogenäther nach Anspruch 1 mit der Formel:

$CCl_3\text{-}CF_2\text{-O-CClF-}CClF_2$.

10. Fluorhalogenäther nach Anspruch 1 mit der Formel:

$CBrF_2\text{-}CF_2\text{-O-CClF-}CClF_2$.

11. Fluorhalogenäther nach Anspruch 1 mit der Formel:

$CF_3\text{-O-}CF_2\ CF_2\text{-O-CClF-}CClF_2$.

12. Fluorhalogenäther nach Anspruch 1 mit der Formel:

$C_2F_5\text{-O-}CF_2CF_2\text{-O-CClF-}CClF_2$.

13. Fluorhalogenäther nach Anspruch 1 mit der Formel:

$(CF_3)_2CF\text{-O-CClF-}CClF_2$.

14. Fluorhalogenäther nach Anspruch 1 mit der Formel:

$CF_3CF_2CF_2\text{-O-CClF-}CClF_2$.

15. Verfahren zur Herstellung der Fluorhalogenäther nach einem der vorgehenden Ansprüche, wonach man eine Fluor-oxy-halogenverbindung mit einem halogenierten Olefin nach dem Reaktionsschema:

$$(R)_nC(F)_m\text{-O-F} + CAF{=}CAF \rightarrow (R)_nC(F)_m\text{-O-CAF-}CAF_2$$

umsetzt, wobei A, R, n und m die oben angeführte Bedeutung haben.

**Patentansprüche** : für den Vertragsstaat AT

1. Verfahren zur Herstellung von Fluorhalogenäther der allgemeinen Formel:

$$(R)_nC(F)_m\text{-O-CAF-}CAF_2$$

wobei A unter Chlor und Brom gewählt ist, R ein alkylischer, cycloalkylischer, aromatischer,

alkylmonoätherischer oder alkylpolyätherischer Rest mit 1 bis 20 Kohlenstoffatomen ist, welcher teilweise oder vollkommen mit Brom, Chlor, Jod und/oder Fluor Substituenten halogeniert ist, n eine der ganzen Zahlen 1 oder 2 und m die ganze Zahl 3-n bedeutet, wobei der Wert n = 2 solche Verbindungen umfasst, in welchen C Bestandteil eines cyclischen Ringes ist, wobei dieses Verfahren darin besteht, dass man eine Fluor-oxy-halogenverbindung mit einem halogenierten Olefin nach dem Reaktionsschema:

$$(R)_nC(F)_m\text{-O-F} + CAF{=}CAF \rightarrow (R)_nC(F)_m\text{-O-CAF-}CAF_2$$

umsetzt, wobei A, R, n und m die oben angeführte Bedeutung haben.

2. Verfahren nach Anspruch 1, wobei R ein perhalogenalkylischer Rest mit 1 bis 5 Kohlenstoffatomen ist.

3. Verfahren nach Anspruch 2, wobei der perhalogenalkylische Rest Halogene, wie Fluor, Chlor und/oder Brom enthält.

4. Verfahren nach Anspruch 1, wobei R ein perfluoralkylmonoätherischer Rest mit bis zu 5 Kohlenstoffatomen ist.

5. Verfahren nach einem der vorangehenden Ansprüche, wobei A Chlor ist.

**Revendications** pour les Etats contractants: BE, CH, DE, FR, GB, LI, NL, SE

1. Fluoro-halo-éthers ayant la formule générale:

$$(R)_nC(F)_m\text{-O-CAF-}CAF_2$$

dans laquelle A est choisi entre chlore et brome, R est un radical alkylique, cycloalkylique, aromatique, alkylmono-éther ou alkyl-poly-éther contenant de 1 a 20 atomes de carbone, partiellement ou complètement halogéné avec du brome, chlore, iode et/ou fluor, n est un nombre entier choisi entre 1 et 2, m est un nombre entier égal 3-n, dans laquelle la valeur n = 2 comprend les composes dans lesquels C appartient à un cycle.

2. Fluoro-halo-éthers suivant la revendication 1, dans lesquels R est un radical per-halo-alkylique contenant de 1 à 5 atomes de carbone.

3. Fluoro-halo-éthers suivant la revendication 2, dans lesquels le radical per-halo-alkylique contient halogènes du type du fluor, chlore et/ou brome.

4. Fluoro-halo-éthers suivant la revendication 1, dans lesquels R est un radical per-fluoro-alkyl-mono-éther contenant jusqu' à 5 atomes de carbone.

5. Fluoro-halo-éther suivant chacune des revendications précédentes, dans lesquels A est chlore.

6. Fluoro-halo-éther suivant la revendication 1, ayant la formule:
$CF_3\text{-}CF_2\text{-O-CClF-}CClF_2$

7. Fluoro-halo-éther suivant la revendication 1, ayant la formule:
$CClF_2\text{-}CF_2\text{-O-CClF-}CClF_2$

8. Fluoro-halo-éther suivant la revendication 1, ayant la formule:
$CCl_2F\text{-}CF_2\text{-O-CClF-}CClF_2$

9. Fluoro-halo-éther suivant la revendication 1, ayant la formule:
$CCl_3\text{-}CF_2\text{-O-CClF-}CClF_2$

10. Fluoro-halo-éther suivant la revendication 1, ayant la formule:
$CBrF_2\text{-}CF_2\text{-O-CClF-}CClF_2$

11. Fluoro-halo-éther suivant la revendication 1, ayant la formule:
$CF_3\text{-O-}CF_2CF_2\text{-O-CClF-}CClF_2$

12. Fluoro-halo-éther suivant la revendication 1, ayant la formule:
$C_2F_5\text{-O-}CF_2CF_2\text{-O-CClF-}CClF_2$

13. Fluoro-halo-éther suivant la revendication 1, ayant la formule:
$(CF_3)_2CF_2\text{-O-CClF-}CClF_2$

14. Fluoro-halo-éther suivant la revendication 1, ayant la formule:
$CF_3CF_2CF_2\text{-O-CClF-}CClF_2$

15. Procédé pour la préparation des fluoro-halo-éthers suivant chacune des revendications précédentes, consistant en réagissant un fluoro-oxy-halo-composé avec une oléfine halogenée suivant le schéma de réaction:

$$(R)_nC(F)_m + CAF{=}CAF \rightarrow (R)_nC(F)_m\text{-O-CAF-}CAF_2$$

dans lequel A, R, n, et m ont le sens précédemment indiqué.

**Revendications** : four l'Etat contractant: AT

1. Procédé pour la preparation de fluoro-halo-éthers ayant la formule générale:

$$(R)_nC(F)_m\text{-O-CAF-CAF}_2$$

dans laquelle A est choisi entre chlore et brome, R est un radical alkylique, cycloalkylique, aromatique, alkylmono-éther ou alkyl-poly-éther contenant de 1 à 20 atomes de carbone, partiellement ou complètement halogéné avec du brome, chlore, iode et/ou fluor, n est un nombre entier choisi entre 1 est 2, m est un nombre entier égal 3-n, dans laquelle la valeur n = 2 comprend les composés dans lesquels C appartient à un cycle, procédé consistant en réagissant un fluoro-oxy-halo-composé avec une oléfine halogenée suivant le schéma de réaction:

$$(R)_nC(F)_m\text{-O-F} + CAF{=}CAF \rightarrow (R)_nC(F)_m\text{-O-CAF-CAF}_2$$

dans lequel A, R, n, et m ont le sens précédemment indiqué.

2. Procédé suivant la revendication 1, dans lesquels R est un radical per-halo-alkylique contenant de 1 à 5 atomes de carbone.

3. Procédé suivant la revendication 2, dans lesquels le radical per-halo-alkylique contient halogènes du type du fluor, chlore est/ou brome.

4. Procédé suivant la revendication 1, dans lesquels R est un radical perfluoro-alkyl-monoéther contenant jusqu'à 5 atomes de carbone.

5. Procédé suivant chacune des revendications précéden es, dans lesquels A est chlore.